# EUROPEAN PATENT SPECIFICATION

(11) **EP 1 494 792 B1**
(45) Date of publication and mention of the grant of the patent: **23.01.2008**
(21) Application number: 02791158.5
(22) Date of filing: 02.12.2002
(51) Int. Cl.: B01F 7/16, A61F 2/46

(54) **MIXING DEVICE FOR THE PREPARATION OF BONE CEMENT**
MISCHVORRICHTUNG ZUR ZUBEREITUNG VON KNOCHENZEMENT
DISPOSITIF MELANGEUR POUR LA PREPARATION DE CIMENT OSSEUX

(30) Priority: 18.04.2002 SE 0201180
(43) Date of publication of application: 12.01.2005
(73) Proprietor: CEMVAC SYSTEM AKTIEBOLAG, 589 41 Linköping (SE)
(72) Inventor: JONSSON, Sören, S-582 70 Linköping (SE)
(74) Representative: Belcher, Simon James
(86) International application number: PCT/SE2002/002208
(87) International publication number: WO 2003/101596

(56) References cited:
- US-A- 5 494 349

## Description

The present invention relates to a device according to the pre-characterising clause of claim 1.

As examples of the prior art, reference will be made to US patents 4,185,072, 4,961,647, 5,494,349 and 5,549,381 and to EP 1 068 893 A2.

In the preparation of bone cement it is extremely important that the two constituents of the cement are well mixed, since this substantially affects the strength of the cement. This means, among other things that every effort should be made to prevent a monotonic mixing process. In the aforementioned examples of the prior art, the drive member operatively connected to the mixing element comprises a manually operatable crank handle. With a drive member in such a form, the possibility cannot be ruled out that the person undertaking the preparation, for reasons of convenience or other reasons, will turn the crank handle round in one direction, which means that the mixing element sweeps over the mixing bowl in one single direction of rotation.

The object of the present invention is to prevent such a monotonic mixing process and this is achieved in that the invention has the features specified in the characterising part of claim 1.

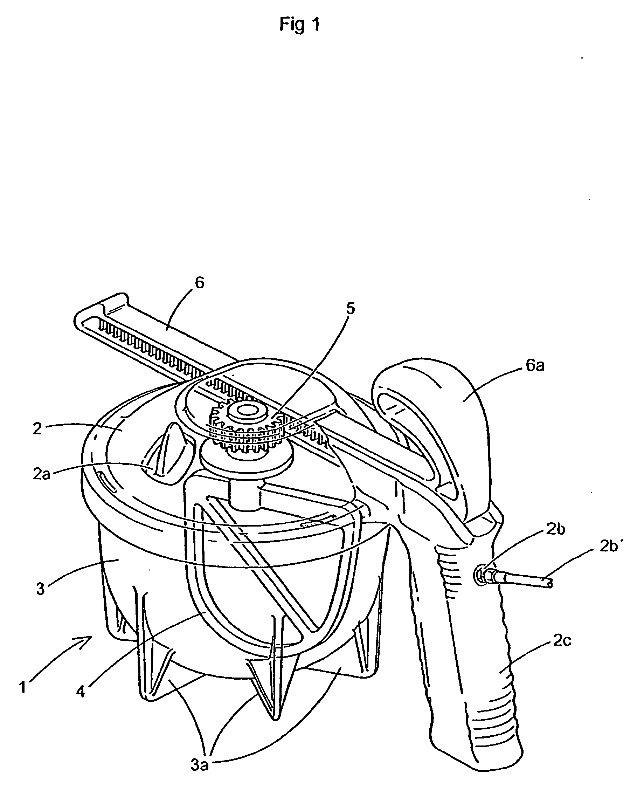

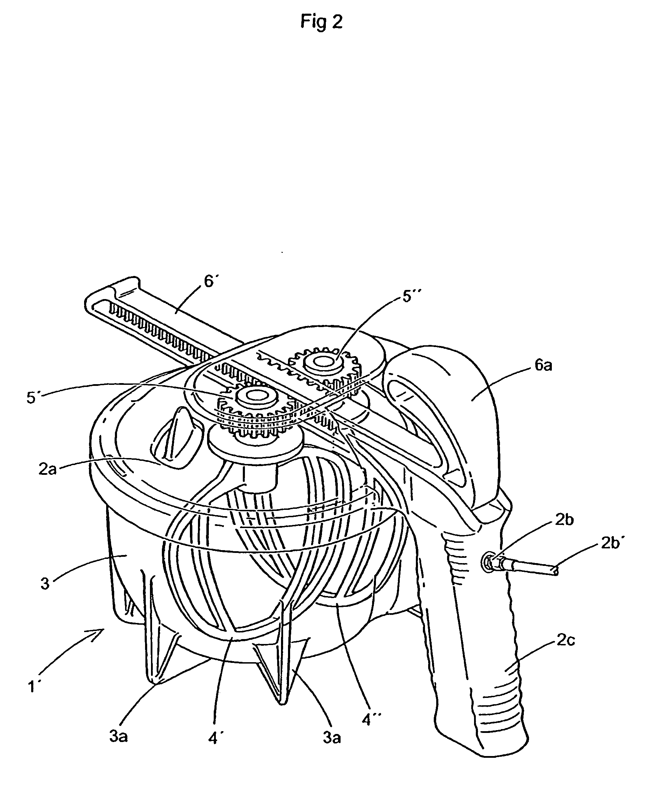

The invention will be explained in more detail below with reference to the drawing attached, in which Fig. 1 shows a perspective view of one embodiment of a mixing vessel according to the invention. Fig. 2 shows a corresponding view of a mixing vessel having two sub-chambers and mixing elements designed to sweep over the said sub-chambers.

In the drawing, 1 generally denotes a mixing vessel intended when in use to be placed on a flat surface, such as a work-table. The mixing vessel comprises a mixing bowl 3 provided with a tightly sealing lid 2. It is intended that the user should grip the lid 2, which is provided with a handle 2c, with one hand when preparing bone cement. In order to achieve good stability, the handle 2c is designed so that in use its remote end rests on the underlying work surface. There are also leg-like supports 3a, intended for the same purpose, projecting radially from the mixing bowl 3.

Rotatably supported in the lid 2 of the mixing vessel 1 is a mixing element 4 designed to sweep over substantially the whole internal chamber of the mixing bowl 3 for the purpose of mixing the two constituents of the bone cement with one another.

One constituent is a polymer in powder form, with which the mixing vessel may be suitably pre-filled, and the other is a liquid monomer, which is delivered to the mixing vessel by way of a sealable aperture 2a in the lid.

Mixing of the bone cement constituents is done under vacuum, partly in order to prevent porous inclusions in the bone cement, and partly in order to prevent noxious gases escaping from the mixing vessel. For this purpose the handle 2c contains a connection aperture 2b, communicating with the interior of the mixing vessel 3, for a vacuum hose 2b'.

The introduction of one or both of the constituents of the bone cement is preferably achieved by means of the vacuum prevailing in the internal chamber of the mixing bowl 3.

In order to enable the mixing element 4, rotatably supported in the lid 2, to perform a rotational movement, the direction of which reliably changes in accordance with a predefined pattern, a toothed gear 5, which meshes with a rack 6, which is displaceably supported in the lid 2 and has a handle 6a, by means of which the rack 6 can be manually displaced forwards and backwards in its longitudinal direction, is connected to the mixing element 4, the mixing element 4 being compelled to perform corresponding rotational movements in opposing directions. In this way an efficient mixing of the bone cement constituents is achieved.

The embodiment according to Fig. 2 differs from the embodiment explained above with reference to Fig. I in that the internal chamber of the mixing bowl 3 comprises two sub-chambers each having a mixing element 4' and 4" respectively, which are rotatably supported in the lid 2 and each have their own toothed gear 5' and 5" respectively meshing with the rack 6'. The latter is provided with teeth on opposite sides.

Since the mixing elements 4', 4" sweep over a common internal chamber, synchronisation of the mixing elements is required so that these do not collide and catch in one another. Such synchronisation is achieved by the toothed gears 5', 5" meshing with the rack 6'. With the two mixing elements 4', 4" it is possible to achieve an improved mixing result.

## Claims

1. A mixing device for the preparation of bone cement, which comprises a mixing bowl (3) provided with tightly sealing lid (2), at least one mixing element (4, 4', 4") which is rotatably supported in the lid (2) and is designed to sweep over substantially the whole internal chamber of the mixing bowl (3) in order to carefully mix the two constituents of the bone cement with one another, a connection to a vacuum source in order to create a vacuum in the chamber of the bowl (3) in connection with the preparation, in which one of the constituents may be situated in the internal chamber prior to preparation and the other of the constituents may be delivered to the said chamber via an aperture in the lid (2) by means of the vacuum prevailing in the internal chamber, the device including a drive member operatively connected to the mixing element, **characterised in that** the drive member comprises a toothed gear (5, 5', 5") which is operatively connected to each mixing element (4, 4', 4"), and which meshes with a corresponding rack (6, 6') which has an operating handle (6a) and is supported so that it can slide in the lid (2).

2. A device according to Claim 1, in which the internal chamber of the mixing bowl (3) comprises two sub-chambers, which are open to one another and in which the device includes two mixing elements (4', 4"), each with an associated toothed gear (5', 5"), the toothed gears meshing with teeth on opposite sides of the rack (6'), each of the sub-chambers being designed to be swept over by one of the mixing elements (4', 4").

3. A device according to Claim 1, in which the operating handle (6a) can be used to displace the rack (6) manually forwards and backwards in its longitudinal direction.

## Patentansprüche

1. Mischeinrichtung zur Bereitstellung eines Knochenzements, umfassend einen Mischbehälter (3), der mit einem dichtenden Deckel (2) versehen ist, wenigstens einem Mischteil (4, 4', 4"), das drehbar an dem Deckel (3) gelagert ist und durch Wischen oder Räumen im wesentlichen der gesamten Innenkammer des Mischbehälters (3) ausgelegt ist, um zwei Bestandteile des Knochenzements miteinander gut zu durchmischen, eine Verbindung zu einer Vakuumquelle, um ein Vakuum in der Kammer des Behälters während der Herstellung zu erzeugen, wobei sich eines der Bestandteile in der Innenkammer vor der Herstellung befinden kann und das andere Bestandteil in die Kammer über eine Öffnung in dem Deckel (2) unter dem Einfluß des in der Innenkammer herrschenden Vakuums eingebracht werden kann, wobei die Einrichtung ein Antriebsteil umfaßt, daß betriebsmäßig mit dem Mischteil verbunden ist, **dadurch gekennzeichnet, daß** das Antriebsteil ein Zahngetriebe (5, 5', 5") umfaßt, das mit dem Mischteil (4, 4', 4") betriebsmäßig verbunden ist und in einem Zahneingriff mit einer entsprechenden Stange (6, 6') steht, die einen Betätigungsgriff (6a) aufweist und derart getragen ist, daß sie in dem Deckel (2) gleiten kann.

2. Einrichtung nach Anspruch 1, bei dem die Innenkammer des Mischbehälters (3) zwei Unterkammern umfaßt, die zueinander offen sind und in denen die Einrichtung zwei Mischteile (4', 4") umfaßt, wobei jedes mit einem zugeordneten Zahngetriebe (5', 5") versehen ist, wobei die Zahngetriebe mit Zähnen an gegenüberliegenden Seiten der Stange (6') in Eingriff stehen, wobei jede Unterkammer dazu ausgelegt ist, durch eines der Mischteile (4', 4") geräumt zu werden.

3. Einrichtung nach Anspruch 1, bei dem der Betätigungsgriff (6a) genutzt werden kann, um die Stange (6) manuell vorwärts und rückwärts in dessen Längsrichtung zu verlagern.

## Revendications

1. Dispositif de mélange pour la préparation de cément osseux, qui comprend un bol de mélange (3) pourvu d'un couvercle d'étanchéité (2), au moins un élément de mélange (4, 4', 4") qui est supporté de façon rotative dans le couvercle (2) et est conçu pour réaliser un balayage sur sensiblement la totalité de la chambre interne du bol de mélange (3) afin de mélanger avec soin les deux constituants du ciment osseux l'un avec l'autre, un raccord à une source de dépression afin de créer un vide dans la chambre du bol (3) en association avec la préparation, dans lequel un des constituants peut être situé dans la chambre interne avant la préparation et l'autre des constituants peut être distribué à ladite chambre par l'intermédiaire d'une ouverture dans le couvercle (2) au moyen de la dépression qui prévaut dans la chambre interne, le dispositif comprenant un élément d'entraînement relié de façon opérationnelle à l'élément de mélange, **caractérisé en ce, que** l'élément d'entraînement comprend une roue dentée (5, 5', 5") qui est reliée de façon opérationnelle à chaque élément de mélange (4, 4', 4") et qui s'engrène avec une crémaillère correspondante (6, 6') qui comporte une poignée de manoeuvre (6a) et est supportée de sorte qu'elle puisse coulisser dans le couvercle (2).

2. Dispositif selon la revendication 1, dans lequel la chambre interne du bol de mélange (3) comprend deux sous-chambres qui sont en communication l'une avec l'autre et dans lequel le dispositif comprend deux éléments de mélange (4', 4"), chacun avec une roue dentée associée (5', 5"), les roues dentées s'engrenant avec des dents sur des côtés opposés de la crémaillère (6'), chacune des sous-chambres étant conçue pour être balayée par un des éléments de mélange (4', 4").

3. Dispositif selon la revendication 1, dans lequel la poignée de manoeuvre (6a) peut être utilisée pour déplacer la crémaillère (6) manuellement vers l'avant et vers l'arrière dans sa direction longitudinale.
